# EUROPEAN PATENT APPLICATION

(11) **EP 1 839 595 A1**
(43) Date of publication of application: **03.10.2007**
(21) Application number: 06711901.6
(22) Date of filing: 18.01.2006
(51) Int. Cl.: A61B 17/068

(54) **STAPLE FOR MEDICAL TREATMENT**

(30) Priority: 19.01.2005 JP 2005012105; 13.12.2005 JP 2005358689
(71) Applicant: MAX CO., LTD., Chuo-ku, Tokyo 103-8502 (JP)
(72) Inventor: HASHIMOTO, Masahiko c/o MAX CO., LTD., Tokyo 103-8502 (JP); YAMAGUCHI, Shigenori c/o MAX CO., LTD., Tokyo 103-8502 (JP)
(74) Representative: Turi, Michael
(86) International application number: PCT/JP2006/300632
(87) International publication number: WO 2006/077878

(57) **Abstract**

A medical staple is provided with a crown portion 1 and needle leg portions 3 bent to a lower side by way of curved portions 2 on both ends thereof. An open wound is narrowed by striking the needle leg portion 3 into the skin by bending intervals between the center portion of the crown portion 1 and the curved portion 2 by a stapler. First notches 6 are formed at a lower face portion of curved portions on both sides bent by the stapler, and second notches 7 are formed at an upper face portion thereof between the first notches 6 of the crown portion 1 and remote from the two first notches 6, 6 uniformly by predetermined distances.

## Description

### Technical Field:

The present invention relates to a medical staple used for suturing a wound.

### Background Art:

Generally, as means for suturing a wound in a surgical operation or the like, other than suture thread, a medical staple is used (refer to JP-B2-2607747). The staple is constituted by a crown portion and needle leg portions bent downward from curved portions on both sides thereof, and the needle leg portions are struck to the skin so as to suture a wound by bending intervals between a center portion of the crown portion and the curved portions to a lower side forcibly by a stapler.

In this way, different from a staple for office use, the medical staple is bent between the center portion of the crown portion and the needle leg portions, the left and right needle leg portions are attracted to each other from outer sides to inner sides to constitute a rectangular shape as a whole.

Further, when the wound is healed to some degree and the staple is not needed, the staple should be removed from the wound. In this case, a remover for removing is used, an upper portion of the center portion of the crown portion is held, lower portions of both sides of the hold portion are pulled up to thereby draw the needle leg portions from the skin to be removed. (refer to JP-B-62-044935)

However, according to the above-described methods of striking and removing, when positions of bending the staple differ from each other on left and right sides in striking the needles, the left and the right skins cannot uniformly be attracted together, the wound is sutured in a state of shifting the wound, a shift may be produced in heights of the skins on both sides of the wound, which is not preferable. Similarly, also in removing the needles, when the hold portion of the center of the crown portion and positions of pulling up the needles on both sides thereof are shifted from each other, there is brought about a drawback that only one of the needle leg portions is drawn and other thereof is not drawn. In this case, the other needle leg portion needs to be drawn out by manual operation, and therefore, time and labor is taken, the skin is damaged considerably, and a pain is inflicted on a patient.

### Disclosure of the Invention

According to one or more embodiments of the invention, there is provided a medical staple capable of piercing left and right needle leg portions of the staple into the skin and drawing out the left and right needle leg portions from the skin uniformly by making positions for folding and pressing the needles in striking the needles and in removing the needles accurate.

In accordance with one or more embodiments of the invention, a medical staple is provided with a crownportion, curvedportions provided at both ends of the crown portion, needle leg portions bent downward from the curved portions, a pair of first notches provided at a lower face thereof, and a second notch provided at an upper face thereof between the pair of first notches.

In accordance with one or more embodiments of the invention, the medical staple may be used for narrowing an open wound by striking the needle leg portions into the skin by bending intervals between the center portion of the crown portion and the curved portions by a stapler.

According to the medical staple of one or more embodiments of the invention, when the needle leg portions are struck to the skin by the stapler, the staple is bent substantially by a right angle from the first notches by pressing down both sides of the crown portion. Therefore, the needle leg portions on both sides are uniformly inserted into the skin, and an excellent suture state is achieved.

Further, when the needle leg portions are removed, the staple is bent in a direction reverse to a direction of bending the first notch from the second notch by moving the second notch portion of the crown portion and an interval between the first notch and the second notch in reverse directions on upper and lower sides relatively. In this way, the both left and right sides are bent uniformly, and therefore, the needle leg portions on both sides are drawn out from the skin in the same state.

In accordance with one or more embodiments of the invention, the second notch may be provided at positions remote from the two first notches uniformly by predetermined distances.

In accordance with one or more embodiments of the invention, the first notches may be provided at positions between the center portion of the crown portion and the curved portions.

In accordance with one or more embodiments of the invention, the second notch may include two notches formed at positions of being opposedly remote from each other by interposing the center portion of the crown portion.

According to the medical staple of one or more embodiments of the invention, the two second notches formed at the positions opposedly remote from each other by interposing a center portion of a length of the crown portion in removing the needle leg portions are ensured to be bent uniformly on left and right sides, and therefore, the needle leg portions on both sides achieve to be drawn out from the skin uniformly.

Further, according to one or more embodiments of the invention, the second notch may include one notch formed at the center portion of the crown portion.

According to the medical staple of one or more embodiments of the invention, a single second notch at the center portion of the length of the crown portion in removing the needle leg portions ensures to be bent uniformly on left and right sides, and therefore, the needle leg portions on both sides achieve to be drawn out uniformly despite a simple structure.

Other aspects and advantages of the invention will be apparent from the following description and the appended claims.

### Brief description of the drawings:

[Fig.1]

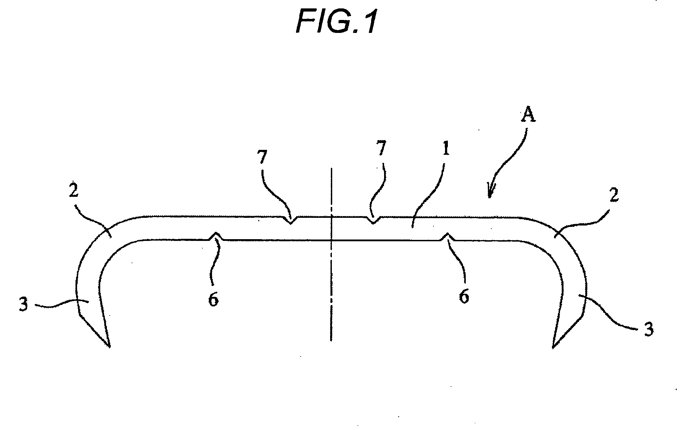

   Fig.1 is a front view of a medical staple according to an embodiment of the invention.
[Fig.2A]

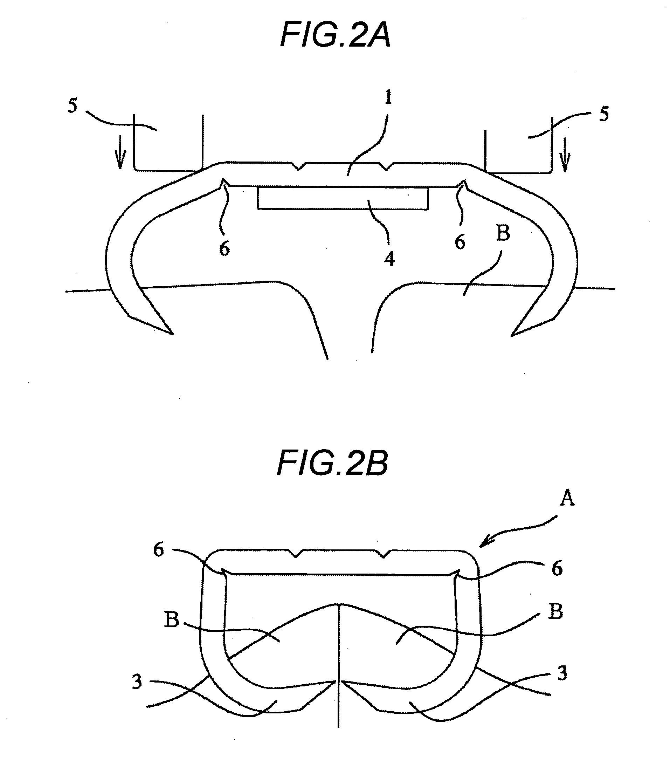

   Fig.2A is an explanatory view showing a shape of the staple of Fig.1 at an initial stage of striking a needle.
[Fig.2B]
   Fig.2B is an explanatory view showing a shape of the staple of Fig.1 in finishing to strike the needle.
[Fig.3A]

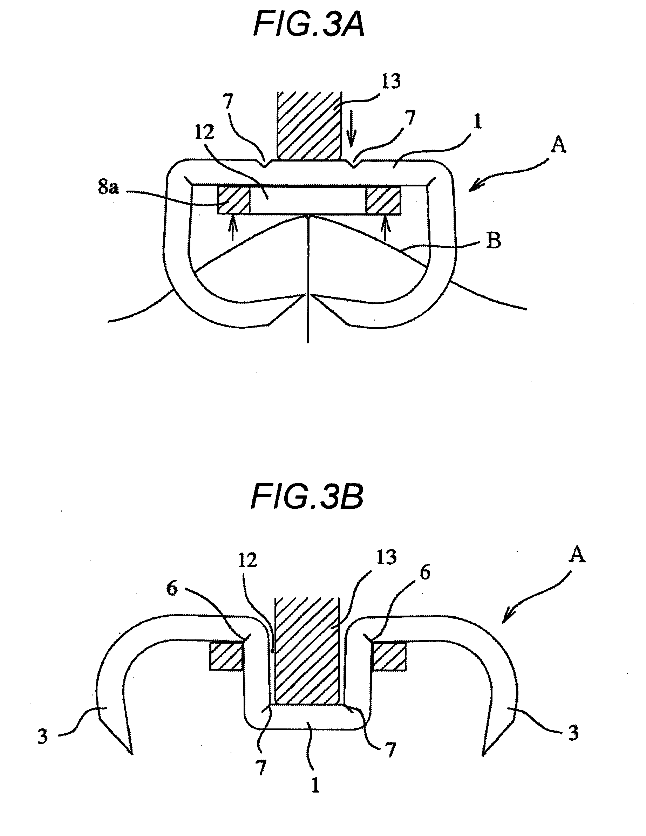

   Fig. 3A is an explanatory view showing a shape of the staple of Fig.1 at an initial stage of removing the needle.
[Fig.3B]
   Fig.3B is an explanatory view showing a shape of the staple of Fig.1 in finishing to remove the needle.
[Fig.4]

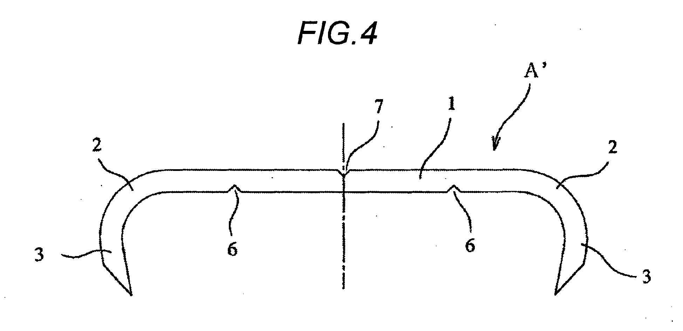

   Fig.4 is a front view of a medical staple according to an embodiment of the invention.
[Fig.5A]
   Fig. 5A is an explanatory view showing a shape of the staple of Fig.4 at an initial stage of removing a needle.
[Fig.5B]

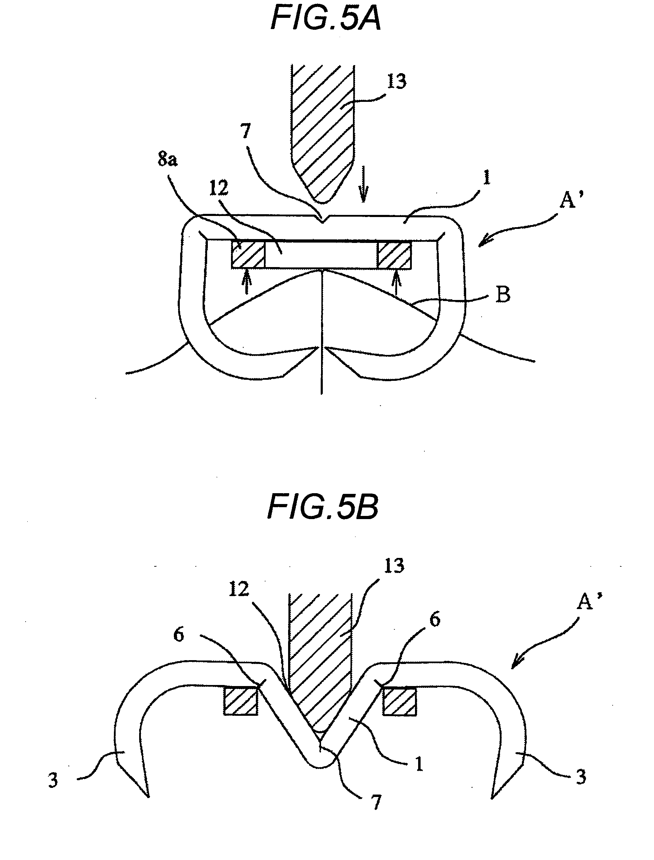

   Fig. 5B is an explanatory view showing a shape of the staple of Fig.4 in finishing to remove the needle.
[Fig.6]

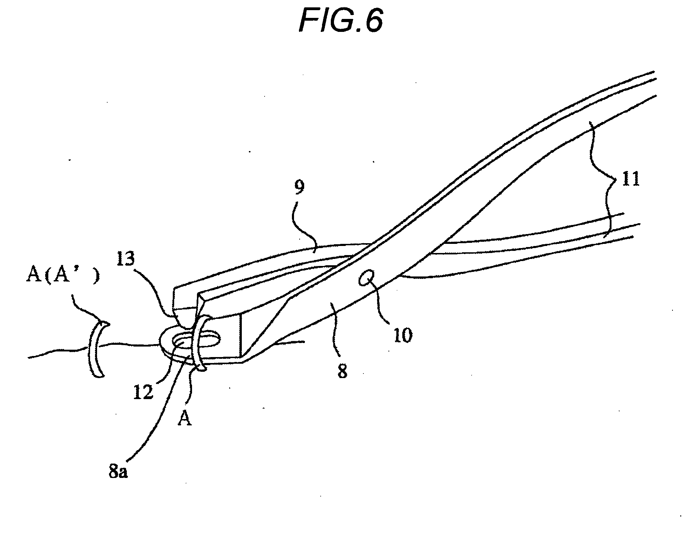

   Fig.6 is a perspective view of a remover.

### Description of Reference numerals and Signs

A, A' .. staples
1..crown portion
3..needle leg portion
6..first notch
7..second notch

### Best Mode for Carrying Out the Invention:

Embodiments of the invention will be explained in reference to the drawings as follows.

### Embodiment 1

In Fig.1, a reference sign A designates a medical staple. The staple A is constituted by a crown portion 1 and a needle leg portion 3 bent downward from curved portions 2 on both sides thereof. A front end of the needle leg portion 3 is formed to be pointed.

When an operation of suturing a wound is carried out by the staple A, a stapler as shown in JP-B2-2607747 is used, as shown by Fig.2A, a lower portion of a center of the crown portion 1 of the staple A is supported by an anvil 4 of the stapler, both sides of the crown portion 1 are folded to bent by being pressed down by a driver plate 5 of the stapler, and the needle leg portions are struck to the skin on both sides of the wound. Hence, in correspondence therewith, first notches 6 are formed at lower face portions of both sides curved portions bent substantially by a right angle by the stapler.

Further, when the staple A is removed from the wound, a remover shown in Fig.6 is used. According to the remover, 2 pieces of arms 8, 9 are axially attached by a shaft 10, portions 11 on one side thereof are made to constitute a grip portion, an opening portion 12 is formed at a front end portion 8a of the arm 8 on one side of portions thereof on other side, and a projected portion 13 capable of being inserted into the opening portion 12 is provided at a front end of the arm 9 on other side, as shown by Fig.3A, a front end of the arm 8 on one side on a side of the opening portion 12 is inserted from below the staple A, and the front end projected portion 13 of the arm on other side is pressed to the opening portion 12 by strongly gripping the grip portion 11. In correspondence therewith, second notches 7 are formed at an upper face portion of the staple A between the center portion of the crown portion 1 and the first notches 6 on both sides thereof.

Further, when the front endprojectedportion 13 is inserted to an inner side of the opening portion 12 of the remover, there is produced a gap to a degree of being slightly larger than a wall thickness of the staple A between both sides of the front end projected portion 13 and the inner face of the opening portion 12.

Further, although it is preferable that the first notches 6 and the second notch 7 of the staple A are formed by a shape of a V groove, the shape may be a shape of a U groove, or may be a cut shape. In sum, the shape may be a shape by which the staple A is easy to be bent from the portion and is not broken by being bent by one time.

Hence, explaining a case of striking the staple and a case of removing the staple by the staple A, first, in striking the needle, when both sides of the crown portion 1 of the staple A are pressed down by the driver plates 5 of the stapler as shown by Fig.2A, as shown by Fig.2B, the staple A is bent substantially by right angles at portions of the first notches 6 on which stresses are concentrated, and therefore, the left and right needle leg portions 3 are attracted to each other uniformly from the outer sides to the inner sides to be struck to the skin B, also both side portions of the wound are attracted to each other to be brought into contact with each other, and a total of the staple A becomes a rectangular shape. In this way, in striking the needles, positions of bending the staple A become the same on left and right sides, and therefore, the skin B on the left and right sides can uniformly be attracted.

Further, when the staple A is removed, as shown by Fig.3A, after inserting the front end of the arm 8 on one side of the remover to between the skin B and the crown portion 1 of the staple A, the remover is gripped strongly, and is moved such that the front end projected portion 13 of the arm on other side is inserted into the opening portion 12 of the arm on one side. Thereby, as shown by Fig. 3B, the two arms are operated relatively in directions reverse to each other in the up and down direction, the front end projected portion 13 is inserted to the opening portion 12, and therefore, also the center portion of the crown portion 1 of the staple A is dragged to be ironed into the opening portion 12 to be bent, simultaneously, the needle leg portions 3 on the both left and right sides are pulled up. At this occasion, stresses are concentrated on the second notches 7 on both sides of the center portion of the crown portion 1, and therefore, the staple A is bent substantially by a right angle by steep angles from the second notches 7 on the both side portions. Further, the first notches 6 on the both left and right sides are engaged with inner edges of the opening portion 12, and therefore, also the needle leg portions 3 on the both sides are pulled up uniformly on the left and right sides, and therefore, the needle leg portions 3 on the both sides are drawn out from the skin B in the same state.

### Embodiment 2

In place of the staple A of the above-described embodiment, a staple A' of the following embodiment illustrated by Fig.4, Fig.5A, and Fig.5B can be constituted.

According to the staple A', the position of bending the needle at the crown portion 1 of the staple A is changed.

Specifically, only a single second notch 7 is formed at the upper face portion of the crown portion 1 bent in removing the needle. The second notch 7 formed as the single notch is formed at a center portion of a length of the crown portion 1, and is constituted by a shape of a V groove, a shape of a U groove, or cut shape or the like similar.to the second notch 7 of the staple A.

The staple A' is struck to the skin by bending the first notches 6 at the lower face portions of the curved portions on the both sides by pressing down the driver plates 5 substantially by the right angle quite similar to the staple A. Therefore, illustration thereof is omitted.

On the other and, the needle of the staple A' is removed by inserting the front end portion 8a of the arm 8 of a remover shown in Fig.6 to between the crown portion 1 of the staple A' and the skin as shown by Fig.5A and Fig.5B, thereafter, gripping the grip portion 11 to thereby press to insert the center of the crown portion 1 to the arm opening portion 12 on one side to move in reverse directions on upper and lower sides relatively similar to needle removing of the staple A, according to the staple A', the portion of the center portion of the crown portion 1 precisely forming the second notch 7 is pressed by the front end projected portion 13 of the arm 9 on other side which is worked to be slender. Further, it is preferable that the front end of the front end projected portion 13 is slightly pointed.

Therefore, the staple A' is bent by the second notch 7 pressed to be inserted to the opening portion 12 of the arm 8 on one side to thereby move in a reverse directions on upper and lower sides relatively by the front end projected portion 13 of the arm 9 on other side by dragging to iron the crown portion 1 in the opening portion 12, whereas according to the staple A, the two notches 7 are bent to thereby fold to bend the crown portion 1 in the shape of the recess groove, simultaneously therewith, the needle leg portions 3 on both left and right sides are pulled up uniformly on left and right sides to be drawn from the skin B, according to the staple A' , the single notch 7 is bent to fold to bend the crown portion 1 in the shape of the V groove, simultaneously therewith, the needle leg portions 3 on both left and right sides are pulled up uniformly on left and right sides to be drawn from the skin B.

Further, in either of the staples A, A' of the two embodiments, in striking and removing the staple, the first notch 6 and the second notch 7 are bent only by once, and therefore, a deterioration in a strength thereof is minimized, and the staple is not broken from the notch portion.

Further, the operation of striking and removing the staple can efficiently be carried out, and the skin is hardly damaged.

Although an explanation has been given of the invention in details and in reference to the specific embodiments, it is apparent for the skilled person that the invention can variously be changed or modified without deviating from the sprit and the range of the invention.

The application is based on Japanese Patent Application (Japanese Patent Application No.2005-012105) filed on January 19, 2005, and Japanese Patent Application (Japanese Patent Application No.2005-358689) filed on December 13, 2005, and a content thereof is incorporated herein by reference.

### Industrial Applicability:

According to one or more embodiments of the medical staple, the left and right needle leg portions 3 can be pierced to the skin and drawn out from the skin uniformly by making positions of bending and pressing the needle in striking the needle and in removing the needle accurate.

## Claims

1. A medical staple comprising:
a crown portion;
curved portions provided at both ends of the crown portion;
needle leg portions bent downward from the curved portions;
a pair of first notches provided at a lower face; and
a second notch provided at an upper face between the pair of the first notches.

2. The medical staple according to Claim 1, wherein the medical staple is used for narrowing an open wound by striking the needle leg portions into the skin by bending intervals between the center portion of the crown portion and the curved portions by a stapler.

3. The medical staple according to Claim 1, wherein the second notch is provided at positions remote from the first notches uniformly by predetermined distances.

4. The medical staple according to Claim 1, wherein the first notches are provided at positions between the center portion of the crown portion and the curved portions.

5. The medical staple according to Claim 1, wherein the second notch comprises two notches formed at positions opposedly remote from each other by interposing a center portion of the crown portion.

6. The medical staple according to Claim 1, wherein the second notch comprises one notch formed at a center portion of the crown portion.
